# EUROPÄISCHE PATENTANMELDUNG

(11) **EP 1 415 623 A1**
(43) Veröffentlichungstag der Anmeldung: **06.05.2004**
(21) Anmeldenummer: 03021884.6
(22) Anmeldetag: 27.09.2003
(51) Int. Cl.: A61F 2/44

(54) **Implantat für die Anordnung zwischen Wirbeln der Wirbelsäule**

(30) Priorität: 16.10.2002 DE 10248171
(71) Anmelder: Advanced Medical Technologies AG, 66620 Nonnweiler-Braunshausen (DE)
(72) Erfinder: Kast, Erich, Dr.med., 8422 Pfungen (CH); Weiland, Peter, 66620 Nonnweiler-Braunshausen (DE)
(74) Vertreter: Bernhardt, Winfrid, Dr.-Ing.

(57) **Zusammenfassung**

Die Erfindung betrifft ein Implantat für die Anordnung zwischen Wirbeln der Wirbelsäule, wie es insbesondere bei der Verbindung von Wirbeln nach einer Bandscheibenresektion zur Anwendung kommt. Erfindungsgemäß ist eine Formanpassung des Implantats an eine bestehende Vertiefung in den dem Implantat zugewandten Wirbeloberflächen vorgesehen. Diese Anpassung an die geringfügig eingefallene Wirbeloberfläche führt zu einer geringeren Beanspruchung der Wirbelkörper durch das Implantat und sorgt für eine hohe Lagestabilität des Implantats zwischen den Wirbeln.

## Beschreibung

Die Erfindung betrifft ein Implantat für die Anordnung zwischen Wirbeln der Wirbelsäule.

Implantate für die Anordnung zwischen Wirbeln der Wirbelsäule kommen zur Anwendung, wenn nach Resektion einer Bandscheibe die betroffenen Wirbel unter Überbrückung des zwischen ihnen entstandenen Zwischenraums miteinander zu verbinden sind. Hierbei dient das Implantat einerseits als Abstandhalter. Dem mit Durchbrüchen versehenen Implantat kommt ferner eine Verbindungsfunktion zu, indem es von Knochengewebe durchdrungen und eine das Implantat einschließende Knochengewebsverbindung zwischen Wirbeln gebildet wird.

Der Erfindung liegt die Aufgabe zugrunde, ein neues Implantat der eingangs erwähnten Art zu schaffen, das in bezug auf die obengenannten Funktionen verbessert ist.

Das diese Aufgabe lösende Implantat nach der Erfindung ist gekennzeichnet durch eine Formanpassung des Implantats an eine Vertiefung in den dem Implantat zugewandten Wirbeloberflächen.

Erfindungsgemäß wird bei der Form des Implantats berücksichtigt, dass die dem Implantat zugewandten Wirbeloberflächen nicht eben, sondern leicht eingefallen sind. Überraschend führt diese Formanpassung zu einer wesentlichen Verbesserung der Lagestabilität des Implantats zwischen den Wirbeln. Ferner werden hohe Flächenpressungen und damit Beanspruchungen des Knochengewebes durch das Implantat vermieden. Beide Faktoren fördern ein schnelles und problemloses Wachstum des Verbindungsknochengewebes unter Durchdringung des Implantats.

Eine solche Formanpassung kann gemäß einem Ausführungsbeispiel für die Erfindung darin bestehen, dass in Richtung von der Vorder- zur Rückseite der Wirbelsäule die Höhe des Implantats bis zu einem Maximum ansteigt und wieder abnimmt, wobei entsprechend des Vertiefungsprofils der Wirbeloberfläche das Maximum vorzugsweise im letzten Drittel der betreffenden Implantatlänge liegt.

Eine noch weitergehend komplementäre Gegenfläche zur Wirbeloberfläche weist das Implantat auf, wenn ferner in Richtung senkrecht zu einer die Wirbelsäule von vorn nach hinten durchstoßenden Mittelachse die Höhe des Implantats zu der Mittelachse hin ansteigt. Diese Formanpassung berücksichtigt, dass die Wirbeloberfläche etwa die Form eines Schrögdachs mit einer bis zu dem Maximum ansteigenden und dann wieder abfallenden Firstlinie aufweist.

Vorzugsweise ist das Implantat entsprechend der Symmetrie der einander zugewandten Wirbeloberflächen in bezug auf eine die Wirbelsäulenlängsachse senkrecht schneidende Ebene symmetrisch ausgebildet.

In einer besonders bevorzugten Ausführungsform der Erfindung ist das Implantat zur Anordnung in einem Halbraum des Zwischenraums zwischen den Wirbeln gemeinsam mit einem zweiten, zu dem Implantat spiegelsymmetrischen solchen Implantat vorgesehen. Vorteilhaft lässt sich jedes dieser Implantate dann von der Rückenseite her durch den Nervenkanal hindurch unter Vorbeiführung am Nervenstrang der Wirbelsäule in den Zwischenraum zwischen den Wirbeln einführen. Ein den gesamten Wirbelzwischenraum ausfüllendes solches Implantat könnte nur von vorn eingesetzt werden.

In weiterer Ausgestaltung der Erfindung kann das Implantat Vorsprünge für die Arretierung am Knochengewebe der Wirbel aufweisen, wobei diese Vorsprünge zur weiteren Stabilisierung der Lage des zwischen den Wirbeln angeordneten Implantats beitragen.

Zweckmäßig sind solche Arretierungsvorsprünge in größtmöglicher Entfernung vom Nervenkanal oder/und der Hauptbelastungsachse der Wirbelsäule angeordnet. Mit dem Eindringen der Arretierungsvorsprünge in das Knochengewebe besteht somit weder die Gefahr der Verletzung von Nervenbahnen noch einer Beeinträchtigung der Wirbelsäulenstabilität.

In einer bevorzugten Ausführungsform der Erfindung sind solche Arretierungsvorsprünge durch eine Zahnung gebildet.

In weiterer vorteilhafter Ausgestaltung der Erfindung kann eine in Implantationsrichtung vordere Stirnfläche des Implantats nach vorn ausgewölbt sein. Eine solche Auswölbung erleichtert die Einführung des Implantats in den Zwischenraum zwischen den Wirbeln. In der bevorzugten Ausführungsform der Erfindung ist das Implantat in der Art eines Käfigs hohl mit Wanddurchbrüchen ausgebildet.

In weiterer vorteilhafter Ausgestaltung der Erfindung ist das Implantat in der Draufsicht rahmenartig mit einem zur Ober- und Unterseite des Implantats offenen Durchbruch als Rahmenöffnung ausgebildet. In den Durchbruch kann von der Ober- und Unterseite her Verbindungsknochengewebe einwachsen, wobei auch eine Durchdringung von Öffnungen in den Seiten des Implantats unter weitgehender Einbettung des Implantats in Knochengewebe erfolgen kann.

In der bevorzugten Ausführungsform der Erfindung besteht das Implantat aus einem Kunststoff, vorzugsweise Polyetheretherketon (PEEK). Im Unterschied zu metallischen Werkstoffen ist das Kunststoffmaterial ähnlich nachgiebig wie Knochengewebe und fügt sich daher besser als Metall organisch in das Knochengerüst ein.

Die Erfindung soll nun anhand eines Ausführungsbeispiels und der beiliegenden, sich auf dieses Ausführungsbeispiel beziehenden Zeichnungen näher erläutert werden. Es zeigen:
- Fig. 1: ein erfindungsgemäßes Implantat in einer perspektivischen Darstellung,
- Fig. 2: das Implantat von Fig. 1 in einer Draufsicht,
- Fig. 3: das Implantat von Fig. 1 in einer Längsseitendarstellung,
- Fig. 4: das Implantat von Fig. 1 in einer Stirnseitendarstellung,
- Fig. 5: das Implantat von Fig. 1 in einer geschnittenen Draufsicht,
- Fig. 6: das Implantat von Fig. 1 in einer geschnittenen Stirnseitendarstellung, und
- Fig. 7: Implantate gemäß Fig. 1 im implantierten Zustand zwischen Wirbeln der Wirbelsäule.

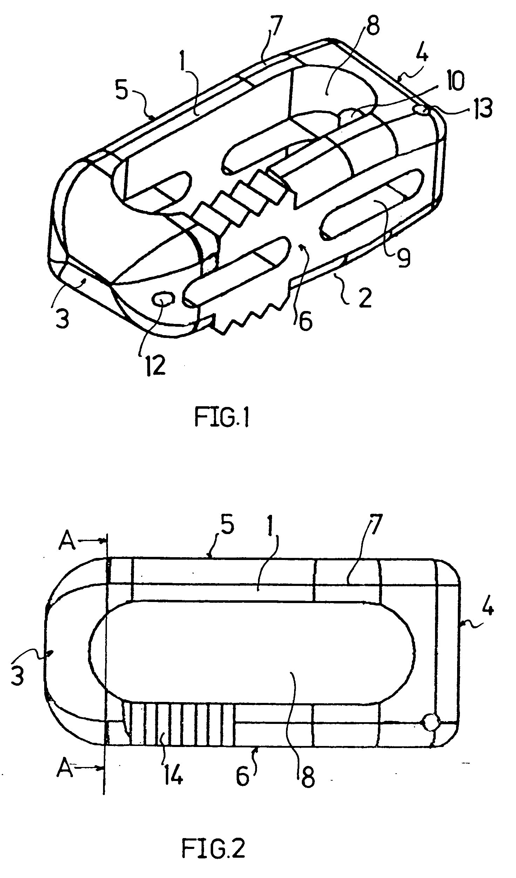

Das in den Fig. 1 bis 7 gezeigte Implantat weist in seinen Umrissen die Form eines länglichen Quaders mit einer Oberseite 1, einer Unterseite 2, Stirnseiten 3 und 4 sowie Längsseiten 5 und 6 auf.

Die Stirnseite 3 des an seinen Ecken und Kanten abgerundeten Quaders ist vorgewölbt, während die der Stirnseite 3 gegenüberliegende Stirnseite 4 eben ist.

In weiterer Abweichung von der Quaderform nimmt die Höhe des Implantats in Längsrichtung von der Stirnwand 3 an zu, erreicht bei 7 ein Maximum und fällt in dem gezeigten Ausführungsbeispiel bis zur Stirnseite 4 wieder ab. Das Implantathöhenmaximum bei 7 liegt von der Stirnseite 3 aus gesehen im letzten Drittel der Länge des Implantats.

Schließlich weicht das Implantat in seinen Umrissen von der Quaderform noch dadurch ab, dass sich die Implantathöhe quer zur Längsrichtung von der Längsseite 5 zur Längsseite 6 hin verringert. In diesem Ausführungsbeispiel beträgt bei einer Maximalhöhe des Implantats von 9,5 mm diese Höhenabnahme ca. 2 mm.

In dem Bereich von der Stirnseite 3 bis zum Implantathöhenmaximum bei 7 sind die Oberseite 1 und die Unterseite 2 des Implantats bezogen auf dessen Längsachse zueinander um 6° geneigt. Die entsprechende Neigung im Bereich zwischen dem Implantatmaximum und der Stirnseite 4 beträgt 16°.

In Bezug auf vertikale Ebenen besteht also keine Symmetrie. Das Implantat ist jedoch zu einer horizontalen Ebene symmetrisch, welche das Implantat in dessen Höhenmitte schneidet.

Wie aus den Fig. 1 bis 7 hervorgeht, weist das Implantat einen zur Oberseite 1 und Unterseite 2 öffnenden vertikalen Durchbruch 8 auf, welcher das Implantat in den Draufsichten von Fig. 2, 5 und 7 rahmenartig aussehen lässt, wobei der vertikale Durchbruch 8 die Rahmenöffnung bildet. Wie den Figuren zu entnehmen ist, weist der vertikale Durchbruch 8 die Form eines an seinen Enden gerundeten Langlochs auf.

An den Längsseiten 5 und 6 sind jeweils zwei Durchbrüche 9 gebildet, welche zu dem vertikalen Durchbruch 8 hin öffnen und wie dieser die Form eines Langlochs mit gerundeten Enden aufweisen.

An der Stirnseite 4 ist eine zu dem vertikalen Durchbruch 8 öffnende Bohrung 10 gebildet. Diese Bohrung 10 weist eine Einsenkung in Form eines Schlitzes 11 mit sich diametral zur Bohrungsöffnung erstreckenden Schlitzabschnitten auf.

Mit den Bezugszeichen 12 und 13 sind Durchgangsbohrungen mit zueinander senkrechten Bohrungsachsen bezeichnet. Die Bohrungen dienen zur Aufnahme von im Röntgenbild gut sichtbaren Metallstiften, insbesondere Titanstiften.

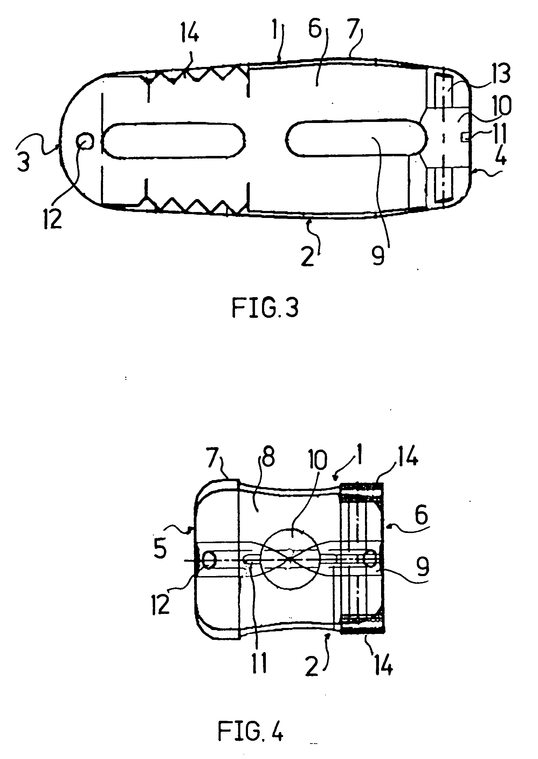

Angrenzend an die Längsseite 6 ist an der Oberseite 1 und der Unterseite 2 des Implantats jeweils eine Zahnung 14 gebildet, deren Zahnkämme sich durchgehend von der Längsseite 6 bis zu dem vertikalen Durchbruch 8 erstrecken. Wie insbesondere Fig. 3 zu entnehmen ist, entspricht der Abstand zwischen den Zahnkämmen einander entsprechender Zähne an der Unter- und Oberseite des Implantats der jeweiligen Implantathöhe auf der gegenüberliegenden Längsseite 5.

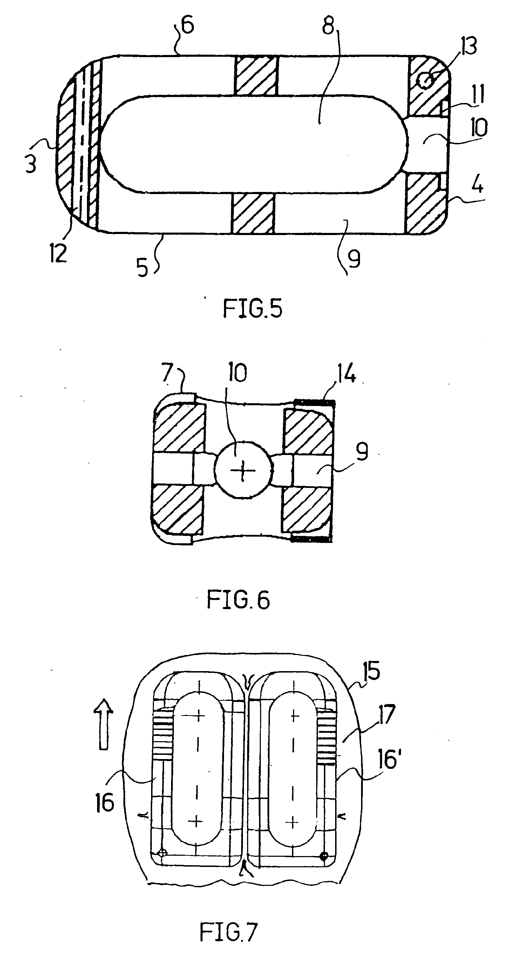

Im folgenden wird die Funktion des vorangehend beschriebenen Implantats unter Bezugnahme auf Fig. 7 erläutert.

Fig. 7 zeigt zwei zwischen Wirbeln 17 der Wirbelsäule 15 angeordnete Implantate 16 und 16', von denen das Implantat 16 mit dem vorangehend anhand der Fig. 1 bis 6 beschriebenen Implantat übereinstimmt. Das Implantat 16' ist zu dem Implantat 16 spiegelsymmetrisch.

Die Einführung der Implantate in den Zwischenraum zwischen den Wirbelkörpern erfolgt in Richtung des in Fig. 7 eingezeichneten Pfeils unter Vorbeiführung des Implantats am Nervenstrang im Nervenkanal (nicht gezeigt) der Wirbelsäule 15. Die Wölbung der Stirnseite 3 erleichtert das Eindringen der Implantate in den Zwischenraum zwischen den Wirbelkörpern, die während der Implantation zur Freihaltung dieses Zwischenraums durch geeignete Halter abzustützen sind.

Zur Einführung des Implantats 16 bzw. 16' in den Zwischenraum zwischen den Wirbeln kann das Implantat durch ein Werkzeug bewegt werden, welches durch die Bohrung 10 hindurch in das Implantat eingreift und für das der Schlitz 11 einen Sitz bildet, der Implantat und Werkzeug gegen Verdrehung zueinander sichert.

In der in Fig. 7 gezeigten Stellung lässt sich durch die Bohrung 10 hindurch ein Schabwerkzeug für die Abtragung von Hartknochengewebe im Bereich des vertikalen Durchbruchs an den dem Implantat zugewandten Knochenoberflächen der Wirbel einführen. Die den vertikalen Durchbruch umgebenden Teile des Implantats liegen dann gegen Hartknochengewebe an, während vor allem das freigelegte Weichknochengewebe neues Knochengewebe bildet, das in den horizontalen Durchbruch eindringen kann und schließlich zur Verbindung der beiden an die Implantate 16,16' angrenzenden Wirbel durch Knochengewebe führt. Auch durch die horizontalen Durchbrüche 9 kann neu gebildetes Knochengewebe dringen, so dass das Implantat weitgehend in die Wirbel verbindendes Knochengewebe eingebettet ist.

Die Zahnung 14 sorgt für eine Arretierung der Implantate 16,16' zwischen den Wirbeln der Wirbelsäule 15, indem die Zähne der Zahnung 14 in das Hartknochengewebe eindringen. Die Zahnung 14 ist in Einführungsrichtung der Implantate gesehen in der ersten Hälfte an der der Außenseite der Wirbelsäule 15 zugewandten Seite des Implantats angeordnet und liegt damit weit vom Nervenstrang der Wirbelsäule und weit von deren Hauptbelastungsachse entfernt. Durch das Eindringen der Zähne in das Knochengewebe kann es daher weder zu einer Beeinträchtigung von Nervenbahnen noch der Belastbarkeit der Wirbelsäule kommen.

Ein besonderer Vorteil der beschriebenen Implantate 16,16' liegt in ihrer Anpassung an die Form der den Implantaten zugewandten Oberfläche der miteinander zu verbindenden Wirbel. Dieser Form wird zum einen dadurch Rechnung getragen, dass das Implantat bei 7 ein Maximum der Höhe aufweisen. Dieses Maximum entspricht einem Maximum der Tiefe der Wirbeloberflächen an der betreffenden Stelle. Quer zur Einführungsrichtung nimmt die Tiefe der Wirbeloberfläche zu den Seiten hin ab. Dem trägt die Abnahme der Implantathöhe von der Längsseite 5 zur Längsseite 6 hin Rechnung.

Durch die weitgehende Formanpassung des Implantats an die Oberfläche des Wirbelkörpers verringert sich die Beanspruchung der miteinander zu verbindenden Wirbel durch das Implantat. Ferner trägt diese Formanpassung zur Lagestabilität des Implantats zwischen den Wirbeln bei. Beide Faktoren fördern letztlich ein schnelles Wachstum des die Wirbel miteinander verbindenden Knochengewebes und damit den Heilungsprozeß nach einer Bandscheibenresektion. Die Anforderungen an die Festigkeit von während des Verbindungsgewebewachstums erforderlicher Stützeinrichtungen verringern sich.

In dem gezeigten Ausführungsbeispiel ist die Implantathöhe an den Stirnenden 3 und 4 gleich groß. Abweichend hiervon könnte die Höhe am Stirnende 4 größer als am Stirnende 3 sein, so dass sich ohne den Anstieg und Abfall der Implantathöhe zwischen den Stirnenden die Gesamtform eines stumpfen Keils ergäbe. Solche in ihrer Grundform keilförmigen Implantate kommen vor allem für den unteren lumbalen Bereich der Wirbelsäule in Betracht, wo die Wirbel im Normalfall zueinander geneigt sind.

Anstelle zweier, in einem Wirbelzwischenraum nebeneinander anzuordnender solcher Implantate ließe sich auch ein aus den beiden Implantaten zusammengesetztes, einstückiges Implantat verwenden, das zu den Seitenrändern dachartig abfällt. Hierbei käme jedoch nur eine Implantation von der Vorderseite der Wirbelsäule her in Betracht.

Anhand der in die Durchgangsbohrungen 12 und 13 eingeführten Metallstifte lässt sich im Röntgenbild genau die Lage der Implantate und damit ihre ordnungsgemäße Implantation überprüfen.

Bei dem verwendeten Kunststoff Polyetheretherketon (PEEK) handelt es sich um ein hoch belastbares Material, das gegenüber metallischen Werkstoffen zudem den Vorteil hat, ähnlich nachgiebig wie Knochengewebe zu sein und sich daher problemloser als Metall in das Knochengewebe einfügt.

Je nach Größe und Verwendung innerhalb der Wirbelsäule kann das Implantat variierende Abmessungen aufweisen, wobei insbesondere unterschiedliche Keilwinkel und unterschiedliche Maximalhöhen in Betracht kommen.

Infolge der erforderlichen Variantenvielfalt werden die benötigten Stückzahlen von Implantaten mit gleichen Abmessungen gering bleiben, so dass gegenüber dem zur Herstellung des Implantats durchaus anwendbaren Spritzgussverfahren einer Zerspanungsbearbeitung der Vorzug zu geben ist, wobei Mischformen der Bearbeitung unter weitgehender Vorfertigung durch Spritzgießen denkbar sind.

## Patentansprüche

1. Implantat (16) für die Anordnung zwischen Wirbeln der Wirbelsäule (15), **gekennzeichnet durch** eine Formanpassung des Implantats (16) an eine Vertiefung in den dem Implantat (16) zugewandten Wirbeloberflächen (17).

2. Implantat nach Anspruch 1,
**dadurch gekennzeichnet,**
**dass** in Richtung von der Vorderseite zur Rückseite der Wirbelsäule (15) die Höhe des Implantats (16) bis zu einem Maximum (7) ansteigt und wieder abnimmt.

3. Implantat nach Anspruch 2,
**dadurch gekennzeichnet,**
**dass** das Maximum (7) in Richtung von der Vorderseite der Wirbelsäule zur Rückseite gesehen im letzten Drittel der Implantatlänge liegt.

4. Implantat nach einem der Ansprüche 1 bis 3,
**dadurch gekennzeichnet,**
**dass** in Richtung senkrecht zu einer die Wirbelsäule (15) von vorn nach hinten durchstoßenden Mittelachse die Höhe des Implantats (16) zu der Mittelachse hin ansteigt.

5. Implantat nach einem der Ansprüche 1 bis 4,
**dadurch gekennzeichnet,**
**dass** das Implantat (16) zu einer die Wirbelsäulenlängsachse senkrecht schneidenden Ebene symmetrisch ausgebildet ist.

6. Implantat nach einem der Ansprüche 1 bis 5,
**dadurch gekennzeichnet,**
**dass** das Implantat (16,16') zur Anordnung in einem Halbraum des Zwischenraums zwischen den Wirbeln gemeinsam mit einem zu dem Implantat (16) spiegelsymmetrischen solchen Implantat (16') vorgesehen ist.

7. Implantat nach einem der Ansprüche 1 bis 6,
**dadurch gekennzeichnet,**
**dass** das Implantat (16) Vorsprünge (14) für die Arretierung am Knochengewebe der Wirbel aufweist.

8. Implantat nach einem der Ansprüche 1 bis 7,
**dadurch gekennzeichnet,**
**dass** eine in Implantationsrichtung vordere Stirnfläche (3) des Implantats (16) nach vorn ausgewölbt ist.

9. Implantat nach einem der Ansprüche 1 bis 8,
**dadurch gekennzeichnet,**
**dass** das Implantat (16) in der Art eines Käfigs hohl mit Wanddurchbrüchen (9,10) ausgebildet ist.

10. Implantat nach einem der Ansprüche 1 bis 9,
**dadurch gekennzeichnet,**
**dass** das Implantat (16) in der Draufsicht rahmenartig mit einem zur Ober- und Unterseite des Implantats öffnenden Durchbruch (8) als Rahmenöffnung ausgebildet ist.

11. Implantat nach einem der Ansprüche 1 bis 10,
**dadurch gekennzeichnet,**
**dass** das Implantat einen Kunststoff, vorzugsweise PEEK, aufweist.
